# EUROPEAN PATENT SPECIFICATION

(11) **EP 0 204 215 B1**
(45) Date of publication and mention of the grant of the patent: **11.08.1993**
(21) Application number: 86106925.0
(22) Date of filing: 22.05.1986
(51) Int. Cl.: C07D 235/28, A61K 31/415

(54) **2-[(1H-benzimidazol-2-ylsulfinyl)methyl]-benzenamines**
2-[(1H-Benzimidazol-2-ylsulfonyl)methyl]-benzenamine
[(1H-benzimidazol-yl-2-sulfonyl)méthyl]-2-benzèneamines

(30) Priority: 24.05.1985 US 737843; 01.04.1986 US 845090
(43) Date of publication of application: 10.12.1986
(73) Proprietor: G.D. Searle & Co., Skokie Illinois 60076 (US)
(72) Inventor: Adelstein, Gilbert William, Evanston, IL 60201 (US); Jones, Peter Hadley, Lake Bluff, IL 60044 (US); Yen, Chung-Hwai, Prospect Heights IL 60070 (US)
(74) Representative: Beil, Hans Chr., Dr.

(56) References cited:
- EP-A- 045 200
- EP-A- 080 062
- FR-A- 2 138 884
- GB-A- 2 134 523
- GB-A- 2 161 160
- GB-A- 2 163 747
- US-A- 4 359 465
- Nature, vol. 290, 12. March 1981, page 159
- TIPS, July 1984, page 263

## Description

This invention relates to compounds that are useful in the treatment and prevention of ulcers. More particularly, this invention relates to 2-[(1H-benzimidazol-2-ylsulfinyl) methyl]benzenamines that inhibit gastric acid secretion and which are, therefore, useful in the treatment of peptic ulcers. The compounds of this invention directly inhibit acid secretion by parietal cells of the stomach through inhibition of (H⁺+K⁺)-ATPase. For review, see, e.g., J. G. Spenney, "Biochemical Mechanisms of Acid Secretion by Gastric Parietal Cells," J. Clin. Gastro., 5 (Suppl. 1), 7-15 (1983). In addition, some of the compounds of this invention also exert cytoprotective activity. For review of cytoprotection, see, e.g., U.S. Patent No. 4,359,465.

### (b) Prior Art

Heterocyclylalkylsulfinylbenzimidazoles have been disclosed as gastric acid secretion inhibitors. See U.S. Patent Nos. 4,472,409, 4,394,509, 4,337,257, 4,327,102, 4,255,431, 4,045,564, and 4,045,563; British Patent No. 2,134,523; and German Offenlegungeschrift No. 3,415,971. Some heterocyclylalkylsulfinylbenzimidazoles have also been disclosed as cytoprotective agents. See U.S. Patent No. 4,359,465. The following structure is illustrative of compounds disclosed in these patents: 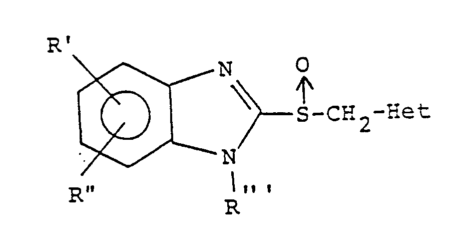
wherein R' and R'' represent hydrogen, alkyl, halogen, trifluoromethyl, cyano, carboxy, hydroxy, acyl, and the like; R''' represents hydrogen, alkyl, acyl, alkoxysulfonyl, and the like; and Het represents heterocyclic groups containing at least one endocyclic (ring) nitrogen. No compound disclosed in these patents includes an unsubstituted or substituted phenyl group instead of the Het group nor exocyclic amino or acylamino functions attached to the Het group. In contrast, the compounds of the present invention are distinguished from the prior art by having (optionally substituted amino)phenyl groups instead of a Het group. Thus, the compounds of the present invention are clearly distinguished from the prior art compounds cited.

Heterocyclylalkylsulfinylnaphth[2,3-d]imidazoles have also been disclosed as gastric acid secretion inhibitors. See U.S. Patent Nos. 4,248,880 and 4,182,766. The compounds disclosed in these patents are related to those illustrated in the above structure, except for having a substituted naphth[2,3-d]imidazole group instead of the benzimidazole group. Similarly, other heterocyclylalkylsulfinylbenzimidazoles having a ring fused to the benzimidazole group have been disclosed as gastric acid secretion inhibitors and cytoprotective agents. See European Patent Application Nos. 130,729 and 127,763. Because of the additional ring fusions of these compounds, as well as for the same reasons stated in the preceding paragraph, the compounds of the present invention are structurally distinguished from prior art compounds cited.

### SUMMARY OF THE INVENTION

The invention relates to compounds of Formula I: 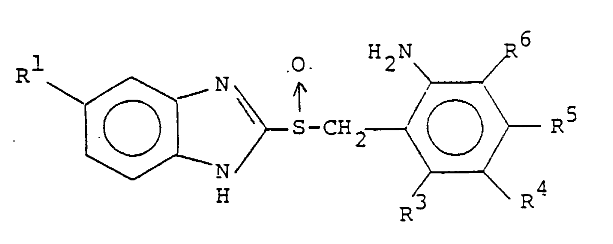
wherein R¹ is hydrogen, methoxy, ethoxy, or trifluoromethyl; and wherein at least one of R³, R⁴, R⁵, and R⁶ is methyl, the others being hydrogen or methoxy, or the pharmaceutically acceptable acid addition salts thereof; or the pharmaceutically acceptable base addition salts thereof.

Although the structure shown for Formula I indicates one tautomeric form, it is understood that this representation is for convenience only and that the scope of this invention includes as equivalents all tautomeric forms of the compounds of this invention.

Examples of pharmacologically acceptable acid addition salts include the hydrochloride, hydrobromide, hydroiodide, sulfate, phosphate, acetate, propionate, lactate, maleate, malate, succinate, and tartrate salts.

Examples of pharmacologically acceptable base addition salts include lithium, sodium, potassium, magnesium, calcium, titanium, ammonium, alkylammomium, dialkylammomium, trialkylammomium, tetraalkylammomium, and guanidinium salts.

### DESCRIPTION OF THE INVENTION

The compounds of this invention may be prepared by the methods illustrated in the following Schemes. Unless otherwise specified, the various substituents are defined as for Formula I, above. Scheme A illustrates the preparation of sulfur-containing intermediates of Formula IV.

### SCHEME A

Intermediates of Formula IV are prepared by at least two routes, each of which uses a 2-mercaptobenzimidazole of Formula II. In the preferred route, the 2-mercaptobenzimidazole reacts with a 2-aminobenzyl halide (Formula III in which Y is a halogen, preferably chlorine or bromine). Typical conditions include reaction at room temperature in an organic solvent such as absolute ethanol or isopropyl alcohol. For those compounds which form hydrohalide salts, the corresponding neutral compounds of Formula IV are readily obtained by methods known to those skilled in the art. For example, treating such a salt with base (such as aqueous potassium carbonate or preferably sodium hydroxide), followed by extraction into a non-protic organic solvent (such as dichloromethane or chloroform), gives the free base form of an intermediate of Formula IV. 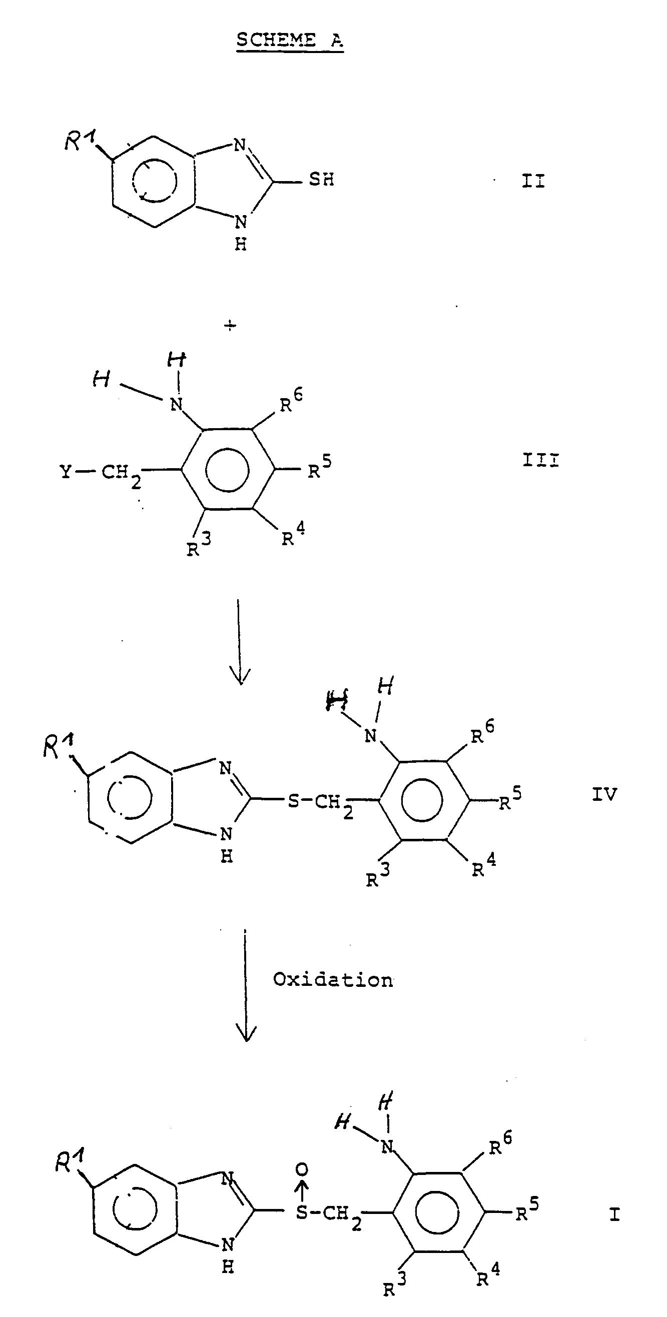
The 2-aminobenzyl halides of (Formula III in which Y is halogen) are obtained commercially or are prepared from corresponding 2-aminobenzyl alcohols (Formula III in which Y is OH) or 2-methylsulfinylmethylanilines (Formula III in which Y is CH₃SO) by synthetic methods well known in the art. For example, reaction of the alcohol with a halogenating reagent, such as thionyl chloride, phosphorus oxychloride, oxalyl chloride, and the like, in an inert organic solvent, such as dichloromethane or chloroform, will give corresponding 2-aminobenzyl chlorides as the hydrochloride salts. A preferred method involves heating the alcohol in concentrated hydrochloric or hydrobromic acid at temperatures of 80° to 100°. See B. Beilenson and F. M. Hamer, J. Chem. Soc., 98-102 (1942).

Where an appropriate 2-aminobenzyl halide of Formula III is not readily available, intermediates of Formula IV may also be prepared by an acid-catalyzed reaction of the 2-mercaptobenzimidazole of Formula II with 2-aminobenzyl alcohols. Preferred conditions include heating a mixture of compounds of Formulas II and III in glacial acetic acid containing excess (relative to II and III) sulfuric acid. After quenching the reaction by pouring the mixture over ice, the intermediate compounds IV are isolated by methods known in the art, including recrystallization and chromatography. Where an initially required 2-aminobenzyl alcohol is not commercially available, corresponding 2-aminobenzoic acids or 2-aminobenzaldehydes may be reduced using methods known in the art, such as hydrogenation, reaction with lithium aluminum hydride, and the like. Various methods for preparing appropriate aminobenzoic acids are known. See, e.g., Baker et al., J. Org. Chem., 17, 141-148, 149-156 (1952). Corresponding 2-nitrobenzyl alcohols or 2-nitrobenzaldehydes may also be reduced using methods known in the art, such as catalytical hydrogenation, to provide the 2-aminobenzyl alcohols.

The sulfoxide compounds of this invention, Formula I, are prepared by oxidation of the intermediates of Formula IV using methods known to those skilled in the art. Commonly used oxidizing agents include, for example, peracids, such as m-chloroperoxybenzoic acid; peresters; peroxides, such as hydrogen peroxide; sodium metaperiodate; selenium dioxide; manganese dioxide; iodosobenzene; and the like. Preferred conditions for preparing sulfoxides of Formula I include oxidizing intermediates IV with an approximately equimolar quantity of m-chloroperoxybenzoic acid in an organic solvent, such as dichloromethane, at temperatures below 0°. Oxidization may be terminated by adding dimethylsulfide.

Certain of the 2-amino compounds of Formula III are more conveniently prepared as phthalimide derivatives (that is, where NH₂ is the phthalimide group) and used to prepare corresponding phthalimide derivatives of compounds of Formula IV, as illustrated in the Examples. Although treatment of such phthalimide derivatives with hydrazine in an alcohol would yield corresponding anilines (that is, compounds of Formula IV where R⁷ and R⁸ are both hydrogen), the preferred next step is oxidation (as described above) to corresponding phthalimide derivatives of sulfoxides of Formula I. Treatment of the phthalimide derivatives of such sulfoxides with hydrazine hydrate in an alcohol, preferably methanol or ethanol, yields compounds of Formula I.

Acid addition salts of this invention may be prepared during the course of the reactions (as described above), by ion exchange from those or other such salts using methods known in the art, or by acidification of free bases of the compounds. Base addition salts of this invention by methods known in the art, including those methods disclosed in British Patent No. 2,137,616.

Although some of the 2-mercaptobenzimidazoles of Formula II are commercially available, others may be prepared by synthetic methods known to those skilled in the art. For example, Scheme B illustrates the preparation of 2-mercaptobenzimidazoles from substituted diaminobenzenes of Formula V.

### SCHEME B

A preferred cyclization method employs an alkali metal alkylxanthate salt of the formula alkyl-O(C=O)S⁻ M⁺, where M⁺ represents an alkali metal ion. Such an alkylxanthate salt may be preformed by methods known in the art or may be formed in situ by mixing an alkali metal hydroxide (preferably sodium hydroxide) and carbon disulfide in an alcohol (preferably ethanol). Preferred cyclization conditions include heating an aqueous or alcoholic mixture of a diaminobenzene of Formula V with sodium or potassium ethylxanthate at reflux under an inert atmosphere, such as argon. 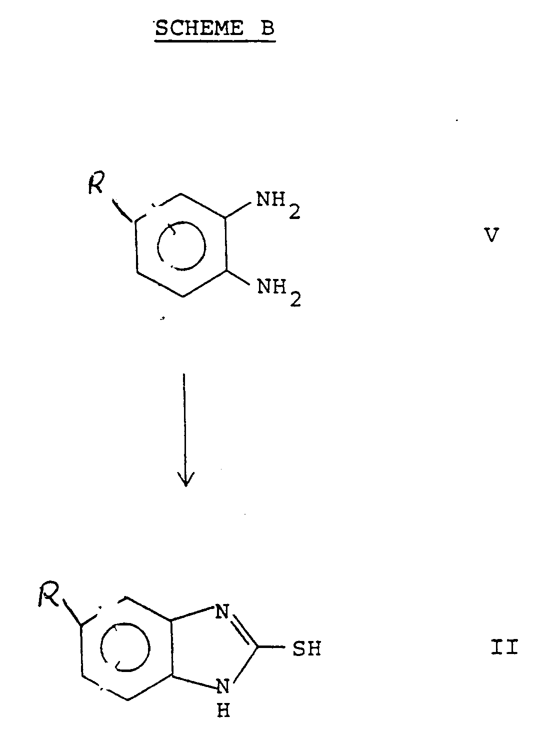
The most preferred embodiments of this invention include compounds of the following general structure,
Formula VII 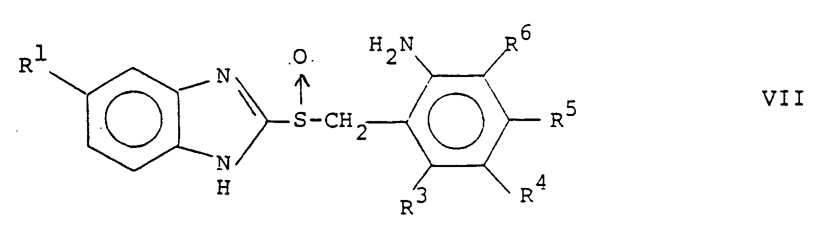
wherein R¹ is methoxy or ethoxy; and wherein R⁴ is methyl, the others being hydrogen.

The compounds of this invention exhibited gastric antisecretory activity in canines, as indicated by inhibition in vitro of (H⁺+K⁺)-ATPase obtained from canine gastric mucosa and by inhibition in vivo of gastric acid secretion in dogs. The antisecretory activity of the compounds of this invention illustrated in the Examples was determined by the following methods.

### Inhibition of (H⁺+K⁺)-ATPase from Canine Gastric Mucosa

Mongrel dogs weighing 15 to 25 kilograms were fasted for twenty-four hours, with water provided ad libitum. The animals were anesthetized with pentobarbital and the stomachs were removed. Subsequent tissue manipulations and subcellular fractionations were performed at 0 to 4°C. After the stomachs were cut open and rinsed with tap water, the antral and cardiac regions were removed and the remaining tissue was rinsed three times in saline. The glandular mucosa was removed mechanically, chopped finely in a medium containing 10 mM Tris hydrochloride (pH 7.4) and 250 mM sucrose, and homogenized. The homogenate was centrifuged at 20,000xg for twenty minutes and the pellet discarded. The supernatant was then centrifuged at 150,000xg for ninety minutes and the supernatant discarded. The pellet was resuspended in the Tris-HCl/sucrose medium by homogenization. Part (2 ml) of the resultant microsomal suspension was layered onto a step gradient consisting of 9 ml of 15% sucrose above 12 ml of 30% sucrose, each sucrose solution being buffered with 10 mM Tris hydrochloride (pH 7.4) containing 0.01% sodium aside. The microsomes retained at the 15%-30% sucrose interface, after centrifugation at 250,000xg for sixty minutes, were used as the source of (H⁺+K⁺)-ATPase. Microsomal preparations were lyophilized, a process that assured potassium ion permiability, and stored at -10° until used.

(H⁺+K⁺)-ATPase activity for each test compound was determined, in duplicate, by measuring the release of inorganic phosphate, which was assayed according to the method of J. ChandraRajan and L. Klein. Anal. Biochem., 72, 407-412 (1976). The (H⁺+K⁺)-ATPase assay medium consisted of 20 mM Mes-Tris (pH 6.0), 5 mM magnesium chloride, 25 mM sucrose, and 4mM Tris-ATP with or without 20 mM potassium chloride in a total volume of 2 ml. Microsomal suspensions (20 to 60 mcl, containing about 25 mcg protein) were added to the assay medium, without Tris-ATP, and then preincubated with a test compound for thirty minutes at 37°. The assay was initiated by adding Tris-ATP and the mixture was incubated another thirty minutes at 37°. A 200-mcl aliquot of the assay mixture was then added to 1.4 ml of a solution consisting of 0.1 M sodium acetate (pH 4.0) and 10% sodium dodecylsulfate, followed by the addition of 200 mcl each of 1% ammonium molybdate and 1% ascorbic acid. At least fifteen minutes later, the optical absorbance at 870 nm (which was proportional to inorganic phosphate concentration up to 100 nmoles per tube, as determined by a standard curve) was obtained. Enzyme activity was linear with incubation time.

(H⁺+K⁺)-ATPase activity is represented by the difference between the measured activities in the presence of potassium ion (K⁺-stimulated) and in the absence of potassium ion (basal). The concentration of a test compound required to inhibit 50% of the (H⁺+K⁺)-ATPase activity (i.e., the IC₅₀) was determined at least in duplicate using linear regression analysis of results obtained for three different compound concentrations ranging from 0.1 mcM to 0.2 mM. If the IC₅₀ for a test compound could not be determined for the concentration range tested, percent inhibition of (H⁺+K⁺)-ATPase was obtained for the compound at 0.1 mM.

### Inhibition of Gastric Acid Secretion in Gastric Fistula Beagle Dogs

Adult female beagle dogs weighing 6 to 11 kilograms obtained from Laboratory Research Enterprises (Kalamazoo, Mich.) or from Hazelton Research Animals (Cumberland, Va.) were surgically implanted with a simple Thomas-type gastric cannula. After recovery from surgery, the dogs were trained to stand quietly, fully conscious, in Pavlov-type dog restraining slings and were acclimated to intravenous infusion of histamine dihydrochloride. During the course of these studies, no dog was used more than once a week. All dogs were deprived of food, but not water, for 18 hours prior to each assay. Each dog was initially infused with 0.15 M sodium chloride solution at a constant rate of 6.5 mg/hr. The volume of gastric secretions, collected in plastic bottles affixed to the cannula, were measured to the nearest 0.1 ml at 30 minute intervals. One of the following protocols was followed, depending on the route chosen for administration of test compound.

Intravenous dosing: Following a 30-minute basal secretion period, test compounds were administered intravenously (i.v.). At the end of an additional 30 minute period, the saline infusion was replaced with histamine dihydrochloride in saline administered at a rate 15 mcg per kilogram of body weight per hour. Histamine stimulation was maintained for a maximum of four hours during which time gastric secretions were collected every 30 minutes. The pH and titratable acidity were determined for samples from each collection period.

Intragastric dosing: Following a 30-minute basal secretion period, the collection bottles were removed, dosing plugs were inserted, and test compounds were administered intragastrically (i.g.). At the end of a 30-minute drug absorption period, the stomachs were emptied, the collection bottles were reattached, and collections were resumed at 30-minute intervals. Simultaneously, the saline infusion was replaced with a continuous intravenous infusion of histamine dihydrochloride in saline administered for four hours at a rate 15 mcg per kilogram of body weight per hour.

Intraduodenal dosing: Dogs were also equipped with duodenal cannulas for intraduodenal (i.d.) administration of test compounds. Dosing was otherwise performed as described for intragastric dosing.

Data from each protocol were analyzed for three gastric sample variables: volume of gastric juice, acid concentration, and total acid output. Percent inhibition for each four-hour experimental period was determined for each parameter by comparison with 3 to 4 controls in which only food was given. Estimates of ED₅₀'s were determined from dose response curves.

### Inhibition of Gastric Acid Secretion in Meal-Stimulated Pavlov Pouch Dog

Adult female beagle dogs weighing 6 to 10 kilograms were obtained from Laboratory Research Enterprises (Kalamazoo, Mich.) or from Hazelton Research Animals (Cumberland, Va.). Surgical implantation of a Thomas-type gastric cannula into an innervated Pavlov pouch of each dog was performed by the method reported by L. Burrows et al., J. Surgical Res., 4, 147-150 (1964). After recovery from surgery, the dogs were trained to stand quietly, fully conscious, in Pavlov-type dog restraining slings. All dogs were deprived of food, but not water, for 24 hours prior to each assay. Basal acid secretion was measured by collecting from the cannulas for 30 minutes before dosing with a test compound. Solutions of the test compounds in iso-osmotic phosphate buffer (pH 7.4) were given either intravenously or directly into the Pavlov pouch. After 30 minutes the pouch was drained. The dogs were then fed 320 grams of dog food and gastric acid secretions were collected each half hour for four hours. Controls were determined in the same way except that only food (i.e., no test compound) was administered.

Data were analyzed for three different variables: volume of gastric juice, acid concentration, and total acid output. Percent inhibition for the four-hour experimental period was determined for each parameter by comparison with 3 to 4 controls. Estimates of ED₅₀ were determined from dose response curves.

By virtue of their gastric antisecretory activity, the compounds of Formula I are useful in treating ulcers in mammals. A physician or veterinarian of ordinary skill can readily determine whether a subject has ulcers. Regardless of the route of administration selected, the compounds of the present invention are formulated into pharmaceutically acceptable dosage forms by conventional methods known to those skilled in the art. The compounds may be formulated using pharmacologically acceptable acid addition or base addition salts. Moreover, the compounds or their salts may be used in a suitable hydrated form.

The compounds can be administered in such oral dosage forms as tablets, capsules, pills, powders, or granules. They may also be administered intravascularly, intraperitoneally, subcutaneously, or intramuscularly, using forms known to the pharmaceutical art. In general, the preferred form of administration is oral. An effective but non-toxic quantity of the compound is employed in treatment. The dosage regimen for preventing or treating ulcers with the compounds of this invention is selected in accordance with a variety of factors, including the type, age, weight, sex, and medical condition of the patient; the severity of the condition; the route of administration; and the particular compound employed. An ordinarily skilled physician or veterinarian can readily determine and prescribe the effective amount of the drug required to prevent or arrest the progress of the condition. In so proceeding, the physician or veterinarian could employ relatively low doses at first and subsequently increase the dose until a maximum response is obtained. Dosages of the compounds of the invention may be in the range of about 1.0 mcg/kg to 500 mg/kg, preferably in the range of about 10 to 100 mg/kg orally or about 1.0 to 20 mg/kg intravenously.

In the pharmaceutical compositions and methods of the present invention, the foregoing active ingredients will typically be administered in admixture with suitable pharmaceutical diluents, excipients, or carriers (collectively referred to herein as "carrier" materials) suitably selected with respect to the intended form of administration, that is, oral tablets, capsules, elixirs, syrups, and the like, and consistent with conventional pharmaceutical practices. For instance, for oral administration in the form of tablets or capsules, the active drug components may be combined with any oral non-toxic pharmaceutically acceptable inert carrier such as lactose, starch, sucrose, cellulose, magnesium stearate, dicalcium phosphate, calcium sulfate, mannitol, and the like; for oral administration in liquid form, the active drug components may be combined with any oral non-toxic pharmaceutically acceptable inert carrier such as ethanol and the like. Moreover, when desired or necessary, suitable binders, lubricants, disintegrating agents, and coloring agents can also be incorporated in the mixture. Suitable binders include starch, gelatin, natural sugars, corn sweeteners, natural and synthetic gums such as acacia, sodium alginate, carboxymethylcellulose, polyethylene glycol, and waxes. Lubricants for use in these dosage forms include boric acid, sodium benzoate, sodium acetate, sodium chloride, and the like. Disintegrators include, without limitation, starch, methylcellulose, agar, bentonite, guar gum, and the like. Sweetening and flavoring agents and preservatives can also be included where appropriate.

The following examples further illustrate details for the preparation of the compounds of this invention. The invention, which is set forth in the foregoing disclosure, is not to be construed or limited either in spirit or in scope by these examples. Those skilled in the art will readily understand that known variations of the conditions and processes of the following preparative procedures can be used to prepare these compounds. All temperatures are degrees Celsius unless otherwise noted.

### DESCRIPTION OF THE PREFERRED EMBODIMENTS

### Example 1 2-[(1H-benzimidazol-2-ylthio)methyl]-N,N-dimethylbenzenamine

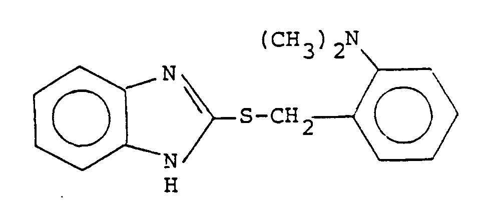

A mixture of 2.20 g (14.6 mmole) of 2-mercaptobenzimidazole and 3.0 g (14.6 mmole) of 2-(chloromethyl)-N,N-dimethylaniline in 120 ml of absolute ethanol were stirred under nitrogen for about two hours. A solid (4.8 g) was collected by filtration, washed with ethanol, and air-dried. The solid was dissolved in water and made basic with potassium carbonate, then extracted into dichloromethane. The organic phase was dried over sodium sulfate, filtered, and concentrated in vacuo to dryness. Recrystallization from acetonitrile gave 2.3 g of a white solid (first crop). Concentration of the acetonitrile liquors gave 0.7 g of a second crop. The two crops were combined and recrystallized from isopropyl alcohol, giving the title compound as an analytically pure solid, m.p. 167-170°.
Analysis. Calcd. for C₁₆H₁₇N₃S: C, 67.81; H, 6.05; N, 14.83; S, 11.31.
Found: C, 67.84; H, 6.19; N, 14.73; S, 11.50.

### Example 2 2-[(1H-benzimidazol-2-ylthio)methyl] benzenamine

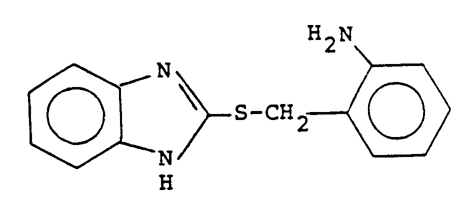

A mixture of 9.0 g (60 mmole) of 2-mercaptobenzimidazole and 4.9 g (40 mmole) of 2-aminobenzyl alcohol were heated at 84° in a mixture of 45 ml of glacial acetic acid and 12.0 g (120 mmole) of sulfuric acid. After two hours an additional 1 g (8 mmole) of 2-aminobenzyl alcohol and 1 g of sulfuric acid were added. After one hour the reaction mixture was cooled and poured into cold (ca. 0°) water containing excess sodium hydroxide. The resultant gummy precipitate was extracted with four portions of dichloromethane. All but the first organic extracts were combined, dried over sodium sulfate, filtered, and concentrated in vacuo. Recrystallization from acetonitrile gave 950 mg of the title compound; column chromatography of the material contained in the liquors on silica gel gave additional title compound, which was recrystallized from acetonitrile to give 1.1 g of the title compound as an analytically pure solid, m.p. 146-148°.
Analysis. Calcd. for C₁₄H₁₃N₃S: C, 65.86; H, 5.13; N, 16.46; S, 12.56.
Found: C, 65.73; H, 5.20; H, 16.63; S, 12.46.

### Example 3 2-[(1H-benzimidazol-2-ylsulfinyl)methyl] benzenamine hemihydrate

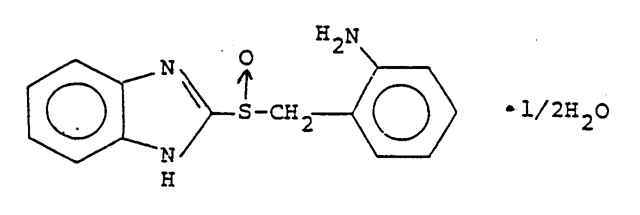

The title product of Example 2 (830 mg, 3.25 mmole) was dissolved in about 200 ml of boiling chloroform and then cooled to about -10°. A solution of 662 mg (ca. 3.25 mmole) of ca. 85% m-chloroperoxybenzoic acid in 10 ml of chloroform was then added with stirring over about ten minutes. After another thirty minutes, the reaction was quenched with 4 drops of dimethylsulfide and a white solid was collected by filtration. The solid was washed sequentially with chloroform and diethyl ether, then air dried, giving 550 mg of the title compound, m.p. 164-165°. Analysis. Calcd. for C₁₄H₁₃N₃OS·1/2H₂O:
C, 59.98; H, 5.03; N, 15.00; S, 11.44.
Found: C, 60.11; H, 4.82; N, 14.93; S, 11.44.

### Example 4 2-[[(5-methoxy-1H-benzimidazol-2-yl)thio] methyl]benzenamine

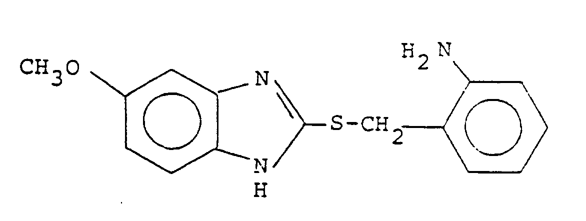

The title compound was prepared by the method of Example 1 using 1.68 g (9.33 mmole) of 2-mercapto-5-methoxybenzimidazole instead of 2-mercaptobenzimidazole and 1.99 g (11.2 mmole) of 2-(chloromethyl)aniline hydrochloride instead of 2-(chloromethyl)-N,N-dimethylaniline in 250 ml of isopropyl alcohol. The basic extraction used 5% sodium hydroxide instead of sodium carbonate. Chromatography on silica gel, followed by crystallization from diethyl ether, gave 520 mg of pure title compound, m.p. 140-142°.
Analysis. Calcd. for C₁₅H₁₅N₃OS: C, 63.14; H, 5.30; N, 14.73; S, 11.23.
Found: C, 63.44; H, 5.42; N, 14.43; S, 11.07.

### Example 5 2-[[(5-methoxy-1H-benzimidazol-2-yl) sulfinyl]methyl]benzenamine

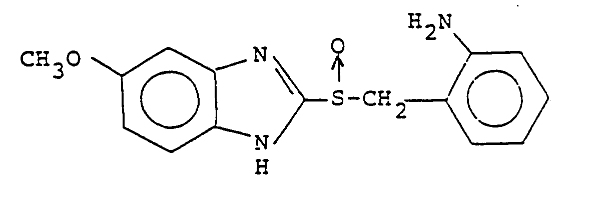

The title compound was prepared by the method of Example 3 using 1.20 g of the title product of Example 11 instead of the title product of Example 2. Trituration with diethyl ether gave 1.04 g of the title compound, m.p. 147-148°.
Analysis. Calcd. for C₁₅H₁₅N₃O₂S: C, 59.78; H, 5.02; N, 13.94; S, 10.64.
Found: C, 59.30; H, 4.95; N, 13.55; S, 10.73.

### Example 6 2-[(1H-benzimidazol-2-ylthio)methyl]-4-methoxybenzenamine dihydrochloride

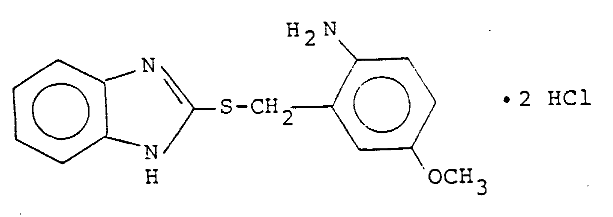

The title compound was prepared by the method of Example 1 using 2.85 g of 2-(chloromethyl)-4-methoxyaniline hydrochloride instead of 2-(chloromethyl)-N,N-dimethylaniline. The precipitate was not neutralized with base but was instead washed sequentially with ethanol and diethyl ether, giving 2.56 g of the title compound as the dihydrochloride, m.p. 206-208°.
Analysis. Calcd. for C₁₅H₁₅N₃OS·2HCl: C, 50.29; H, 4.78; N, 11.73; S, 8.95; Cl, 19.79.
Found: C, 49.95; H, 4.57; N, 11.55; S, 9.07; Cl, 19.09.

### Example 7 2-[(1H-benzimidazol-2-ylsulfinyl)methyl]-4-methoxybenzenamine

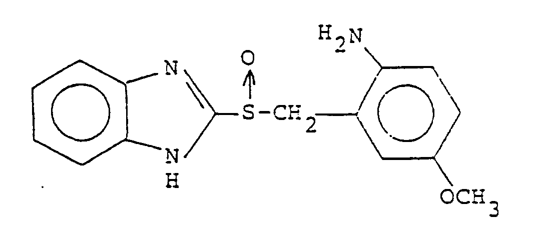

The title compound was prepared by the method of Example 3 using 2.20 g of the free base of the title product of Example 6 instead of the title product of Example 2. Trituration with diethyl ether gave 900 mg of the title compound, m.p. 152-153°.
Analysis. Calcd. for C₁₅H₁₅N₃O₂S: C, 59.78; H, 5.02; N, 13.94; S, 10.64.
Found: C, 59.01; H, 4.97; N, 13.65; S, 10.65.

### Example 8 2-[(1H-benzimidazol-2-ylthio)methyl]-3-methylbenzenamine

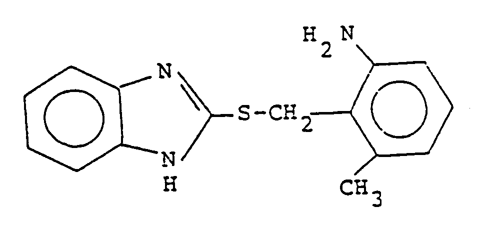

The title compound was prepared by the method of Example 1 using 3.55 g of 2-(chloromethyl)-3-methylaniline hydrochloride instead of 2-(chloromethyl)-N,N-dimethylaniline. Trituration with diethyl ether gave 2.31 g of the title compound.
Analysis. Calcd. for C₁₅H₁₅N₃S: C, 66.89; H, 5.61; N, 15.60; S, 11.90.
Found: C, 66.61; H, 5.53; N, 15.52; S, 11.80.

### Example 9 2-[(1H-benzimidazol-2-ylsulfinyl)methyl]-3-methylbenzenamine 1/4 hydrate

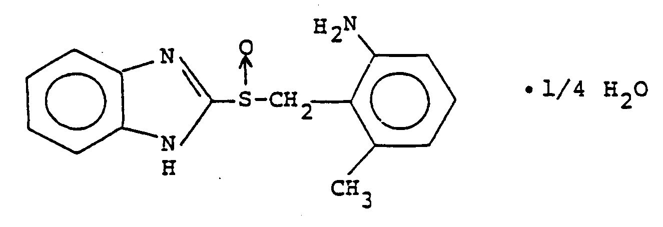

The title compound was prepared by the method of Example 3 using 1.07 g of the title product of Example 8 instead of the title product of Example 2. Trituration with dichloromethane gave 327 mg of the title compound, m.p. 152-153°.
Analysis. Calcd. for C₁₅H₁₅N₃OS·1/4 H₂O: C, 62.21; H, 5.31; N, 14.51; S, 11.07.
Found: C, 62.28; H, 5.05; N, 14.46; S, 11.22.

### Example 10 2-[(1H-benzimidazol-2-ylthio)methyl]-4-methylbenzenamine

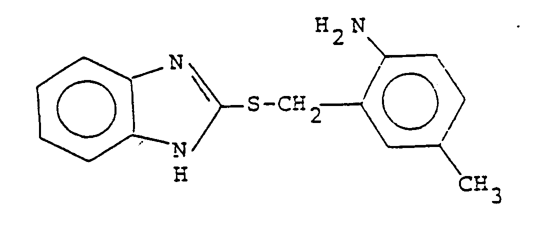

The title compound was prepared by the method of Example 1 using 2-(chloromethyl)-4-methylaniline hydrochloride instead of 2-(chloromethyl)-N,N-dimethylaniline. Chromatography on silica gel gave 650 mg of the title compound.
Analysis. Calcd. for C₁₅H₁₅N₃S: C, 66.89; H, 5.61; N, 15.60; S, 11.90.
Found: C, 66.70; H, 5.65; N, 15.50; S, 11.85.

### Example 11 2-[(1H-benzimidazol-2-ylsulfinyl)methyl]-4-methylbenzenamine

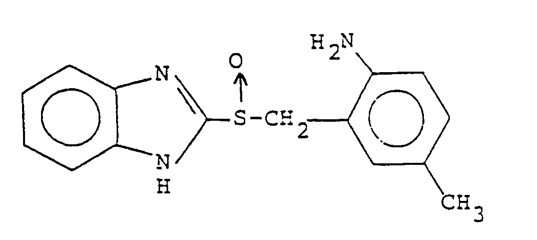

The title compound was prepared by the method of Example 3 using 2.68 g of the title product of Example 10 instead of the title product of Example 2 and using 1,2-dichloroethane as solvent instead of chloroform. Trituration with diethyl ether gave 3.0 g of the title compound.
Analysis. Calcd. for C₁₅H₁₅N₃OS: C, 63.14; H, 5.30; N, 14.73; S, 11.23.
Found: C, 61.44; H, 4.98; N, 14.35; S, 11.10.

### Example 12 2-[(1H-benzimidazol-2-ylthio)methyl]-6-methylbenzenamine

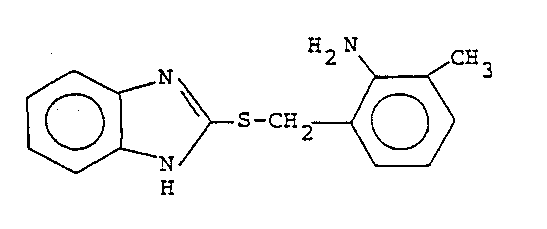

The title compound was prepared by the method of Example 1 using 5.00 g of 2-(chloromethyl)-6-methylaniline hydrochloride instead of 2-(chloromethyl)-N,N-dimethylaniline. Trituration with hexane gave 3.33 g of the title compound, m.p. 130-134°.
Analysis. Calcd. for C₁₅H₁₅N₃S: C, 66.89; H, 5.61; N, 15.60; S, 11.90.
Found: C, 66.85; H, 5.61; N, 15.20; S, 11.50.

### Example 13 2-[(1H-benzimidazol-2-ylsulfinyl)methyl]-6-methylbenzenamine

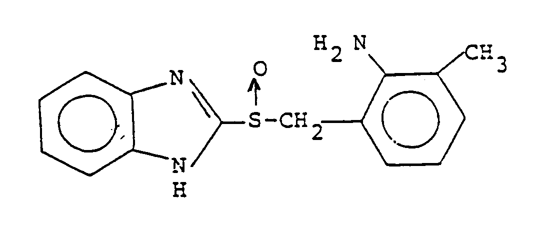

The title compound was prepared by the method of Example 3 using 1.68 g of the title product of Example 12 instead of the title product of Example 2. Trituration with acetonitrile gave 948 mg of the title compound, m.p. 156-157°.
Analysis. Calcd. for C₁₅H₁₅N₃OS: C, 63.14; H, 5.30; N, 14.73; S, 11.23.
Found: C, 62.91; H, 5.18; N, 14.33; S, 11.08.

### Example 14 2-[(1H-benzimidazol-2-ylthio)methyl]-4,6-dimethylbenzenamine

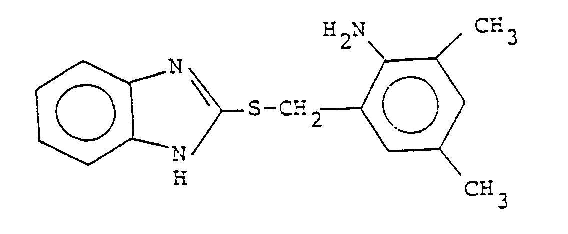

The title compound was prepared by the method of Example 1 using 1.68 g of 2-(chloromethyl)-4,6-dimethylaniline hydrochloride instead of 2-(chloromethyl)-N,N-dimethylaniline. Crystallization from diethyl ether gave 788 mg of the title compound, m.p. 139-141°.
Analysis. Calcd. for C₁₆H₁₇N₃S: C, 67.81; H, 6.05; N, 14.83; S, 11.31.
Found: C, 67.30; H, 5.93; N, 14.66; S, 11.29.

### Example 15 2-[(1H-benzimidazol-2-ylsulfinyl)methyl]-4,6-dimethylbenzenamine hemihydrate

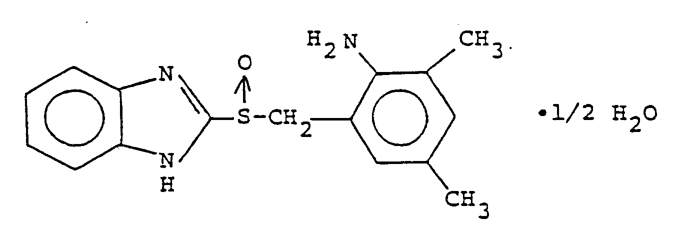

The title compound was prepared by the method of Example 3 using 950 mg of the title product of Example 14 instead of the title product of Example 2. Trituration with diethyl ether gave 434 mg of the title compound.
Analysis. Calcd. for C₁₆H₁₇N₃OS·1/2 H₂O: C, 62.23; H, 5.88; N, 13.63; S, 10.40.
Found: C, 62.39; H, 5.72; N, 13.50; S, 10.65.

### Example 16 2-[[(5-methoxy-1H-benzimidazol-2-yl)thio] methyl]-4-methylbenzenamine

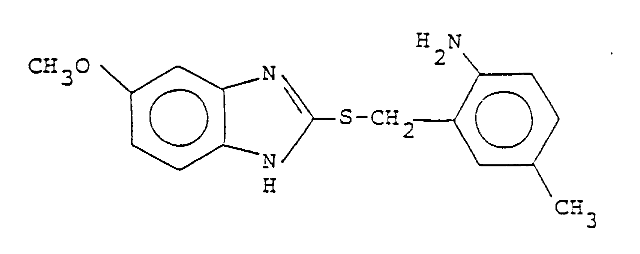

The title compound (1.95 g) was prepared by the method of Example 1 using 2.81 g of 2-mercapto-5-methoxybenzimidazole instead of 2-meraptobenzimidazole and 3.00 g of 2-(chloromethyl)-4-methylaniline hydrochloride instead of 2-(chloromethyl)-N,N-dimethylaniline.
Analysis. Calcd. for C₁₆H₁₇N₃OS: C, 64.19; H, 5.72; N, 14.04; S, 10.71.
found: C, 63.71; H, 5.80; N, 13.86; S, 10.60.

### Example 17 2-[[(5-methoxy-1H-benzimidazol-2-yl) sulfinyl]methyl]-4-methylbenzenamine

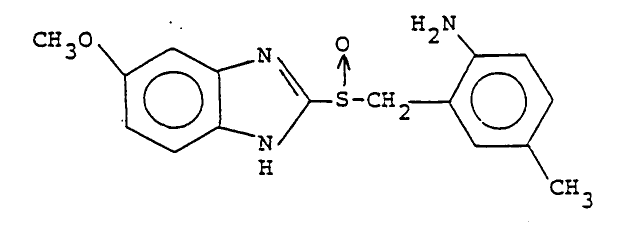

The title compound was prepared by the method of Example 3 using 1.50 g of the title product of Example 16 instead of the title product of Example 2. Concentration to dryness and trituration with diethyl ether gave 1.10 g of the title compound, m.p. 148-149°.
Anaylsis. Calcd. for C₁₆H₁₇N₃O₂S: C, 60.93; H, 5.43; N, 13.32; S, 10.17.
Found: C, 60.67; H, 5.38; N. 13.20; S, 9.95.

### Example 18 2-[[(5-methoxy-1H-benzimidazol-2-yl)thio] methyl]-6-methylbenzenamine

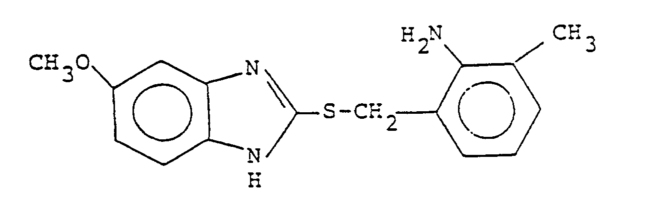

The title compound was prepared by the method of Example 1 using 3.75 g of 2-mercapto-5-methoxybenzimidazole instead of 2-mercaptobenzimidazole and 4.00 g of 2-(chloromethyl)-6-methylaniline hydrochloride instead of 2-(chloromethyl)-N,N-dimethylaniline. Trituration with hexane gave 3.02 g of the title compound, m.p. 132-134°.
Analysis. Calcd. for C₁₆H₁₇N₃OS: C, 64.19; H, 5.72; N, 14.04; S, 10.71.
Found: C, 64.21; H, 5.77; N, 14.00; S, 10.38.

### Example 19 2-[[(5-methoxy-1H-benzimidazol-2-yl) sulfinyl]methyl]-6-methylbenzenamine

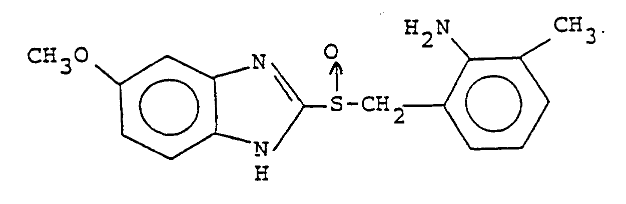

The title compound was prepared by the method of Example 3 using 2.00 g of the title product of Example 18 instead of the title product of Example 2. Trituration with diethyl ether gave 1.58 g of the title compound, m.p. 142-144°.
Analysis. Calcd. for C₁₆H₁₇N₃O₂S: C, 60.93; H, 5.43; N, 13.32; S, 10.17.
Found: C, 60.60; H, 5.42; N, 12.83; S, 9.86.

### Example 20 2-[[[(5-(trifluoromethyl)-1H-benzimidazol-2-yl]thio]methyl]benzenamine

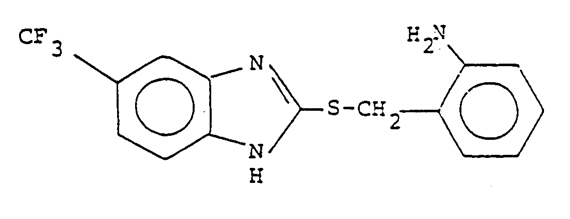

The title compound was prepared by the method of Example 1 using 4.36 g of 2-mercapto-5-(trifluoromethyl) benzimidazole instead of 2-mercapcobenzimidazole and 3.56 g of 2-(chloromethyl)aniline hydrochloride instead of 2-(chloromethyl)-N,N-dimethylaniline in isopropyl alcohol. The basic extraction used 5% sodium hydroxide instead of sodium carbonate. Washing the solid residue from the dichloromethane extract with additional dichloromethane gave 2.40 g of the title compound, m.p. 155-270^{o}.
Analysis. Calcd. for C₁₅H₁₂N₃F₃S: C, 55.72; H, 3.74; N, 13.00; S, 9.92; E, 17.63
Found: C, 55.55; H, 3.68; N, 13.10; S, 10.12; F, 17.38.

### Example 21 2-[[[(5-(trifluoromethyl)-1H-benzimidazol-2-yl]sulfinyl]methyl]benzenamine

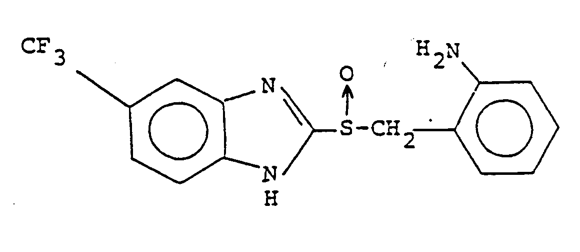

The title compound was prepared by the method of Example 3 using 2.00 g of the title product of Example 20 instead of the title product of Example 2. The reaction mixture, which contained no precipitate, was concentrated in vacuo. Crystallization during the concentration gave 568 mg (in three crops) of the title compound, m.p. 152-152.5^{o}.
Analysis. Calcd. for C₁₅H₁₂N₃F₃OS: C, 53.09; H, 3.56; N, 12.38; S, 9.45; F, 16.80.
Found: C, 53.23; H, 3.61; N, 12.48; S, 9.64; F, 16.89.

### Example 22 2-[(1H-benzimidazol-2-ylthio )methyl]-5,6-dimethylbenzenamine

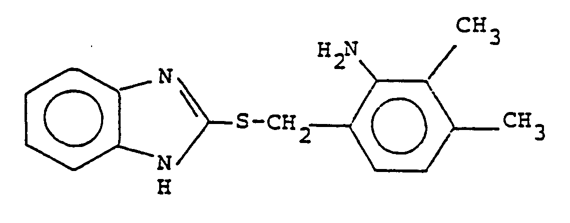

The title compound was prepared by the method of Example 1 using 3.00 g of 2-(chloromethyl)-5,6-dimethylaniline hydrochloride instead of 2-(chloromethyl)-N,N-dimethylaniline in isopropyl alcohol. The basic extraction used 5% sodium hydroxide instead of sodium cardbonate. The solid residue from the dichloromethane extract gave, without further purification, 3.09 g of the title compound.
Analysis. Calcd. for C₁₆H₁₇N₃S: C, 67.81; H, 6.05; N, 14.83; S, 11.31.
Found: C, 67.46; H, 6.36; N, 14.40; S, 10.90.

### Example 23 2-[(1H-benzimidazol-2-ylsulfinyl)methyl]-5,6-dimethylbenzenamine 1/4 hydrate

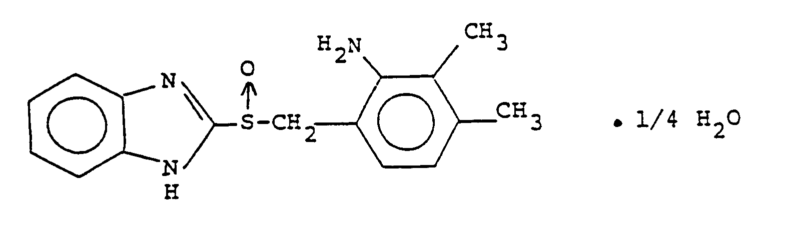

The title compound was prepared by the method of Example 3 using 2.97 g of the title product of Example 22 instead of the title product of Example 2. The reaction mixture was concentrated in vacuo to dryness. The residue was washed with diethyl ether and redissolved in 400 ml of 15% (by volume) of methanol in dichloromethane. The organic solution was washed with 5% aqueous potassium carbonate, dried over sodium sulfate, filtered, and concentrated in vacuo. Crystallization during the concentration gave 1.36 g of the title compound, m.p. 160^{o} (decomp).
Analysis. Calcd. for C₁₆H₁₇N₃OS·1/4H₂O: C, 63.24; H, 5.80; N, 13.85; S, 10.55.
Found: C, 62.72; H, 5.65; N, 13.58; S, 10.50

### Example 24 2-[(1H-benzimidazol-2-ylthio)methyl]-3,6-dimethylbenzenamine

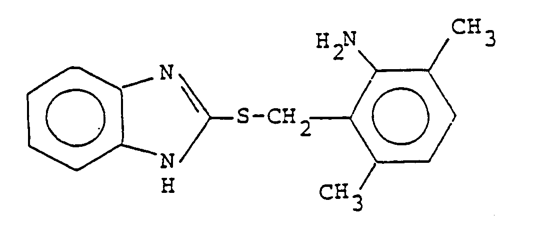

The title compound was prepared by the method of Example 1 using 2.00 g of 2-(chloromethyl)-3,6-dimethylaniline hydrochloride instead of 2-(chloromethyl)-N,N-dimethylaniline in isopropyl alcohol. The basic extraction used 10% sodium hydroxide instead of sodium carbonate. Crystallization during concentration of the dichloromethane extract gave 1.60 g of the title compound.
Analysis. Calcd. for C₁₆H₁₇N₃S: C, 67.81; H, 6.05; N, 14.83; S, 11.31.
Found: C, 67.20; H, 6.03; N, 14.82; S, 11.29

### Example 25 2-[(1H-benzimidazol-2-ylsulfinyl)methyl]-3,6-dimethylbenzenamine monohydrate

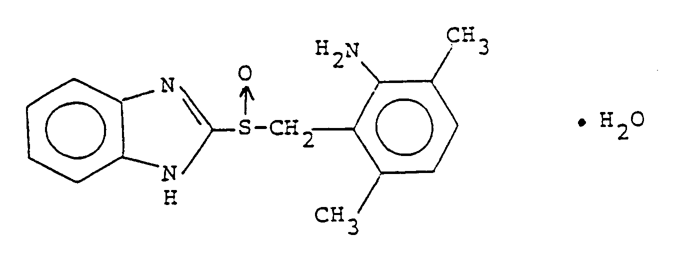

The title compound was prepared by the method of Example 3 using the title product of Example 24 instead of the title product of Example 2. The reaction mixture was concentrated in vacuo to dryness. The residue was washed with diethyl ether and redissolved in 400 ml of 10% (by volume) of methanol in dichloromethane. The organic solution was washed with 10% aqueous potassium carbonate, dried over sodium sulfate, filtered, and concentrated in vacuo. The resultant residue was washed with diethyl ether to give 621 mg of the title compound, m.p. 144-145^{o}.
Analysis. Calcd. for C₁₆H₁₇N₃OS·H₂O: C, 60.55; H, 6.03; N, 13.24; S, 10.10.
Found: C, 60.40; H, 5.69; N, 13.05; S, 9.98.

### Example 26 2-[[(5-ethoxy-1H-benzimidazol-2-yl)thio]-methyl]-4-methylbenzenamine

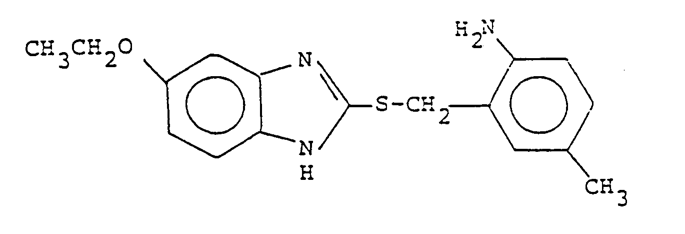

The title compound was prepared by the method of Example 1 using 3.00 g of 2-mercapto-5-ethoxybenzimidazole instead of 2-mercaptobenzimidazole and 3.71 g of 2-(chloromethyl)-4-methylaniline hydrochloride instead of 2-(chloromethyl)-N,N-dimethylaniline in isopropyl alcohol. The basic extraction used 10% sodium hydroxide instead of sodium carbonate. Crystallization during concentration of the dichloromethane extract gave 2.13 g of the title compound, which was used in subsequent reactions without further purification.

### Example 27 2-[[(5-ethoxy-1H-benzimidazol-2-yl)sulfinyl]-methyl]-4-methylbenzenamine

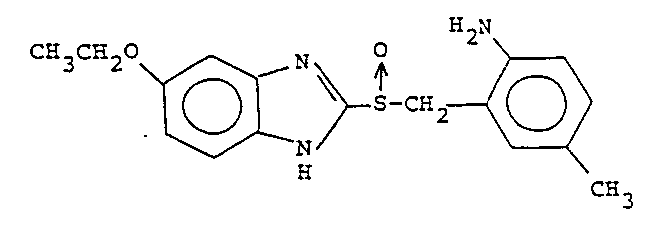

The title compound was prepared by the method of Example 3 using 1.90 g of the title product of Example 26 instead of the title product of Example 2. The reaction mixture, which contained no precipitate, was concentrated in vacuo to dryness. Trituration with diethyl ether gave 1.19 g of the title compound, m.p. 144-145^{o}.
Analysis. Calcd. for C₁₇H₁₉N₃O₂S: C, 61.98; H, 5.81; N, 12.76; S, 9.73.
Found: C, 61.38; H, 5.80; N, 12.57; S, 9.85.

### Example 28 2-[[[(5-(trifluoromethy)1-1H-benzimidazol-2-yl]thio]methyl]-3,6-dimethylbenzenamine

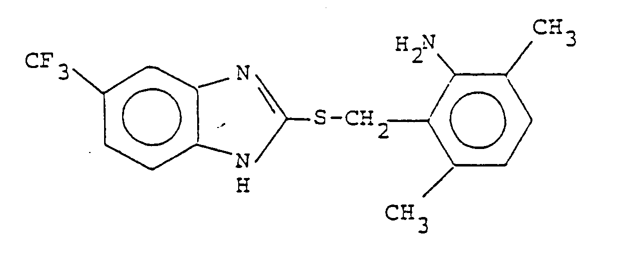

The title compound was prepared by the method of Example 1 using 529 mg of 2-mercapto-5-(trifluoromethyl) benzimidazole instead of 2-mercaptobenzimidazole and 500 mg of 2-(chloromethyl)-3,6-dimethylaniline hydrochloride instead of 2-(chloromethyl)-N,N-dimethylaniline in isopropyl alcohol. The basic extraction used 10% sodium hydroxide in;stead of sodium carbonate. The dichloromethane extract was dried over sodium sulfate, filtered, and concentrated in vacuo to 590 mg of the title compound as a gum. The compound was used in subsequent reactions without further purification.

### Example 29 2-[[[(5-(trifluoromethy)1-1H-benzimidazol-2-yl]thio]methyl]-3,6-dimethylbenzenamine

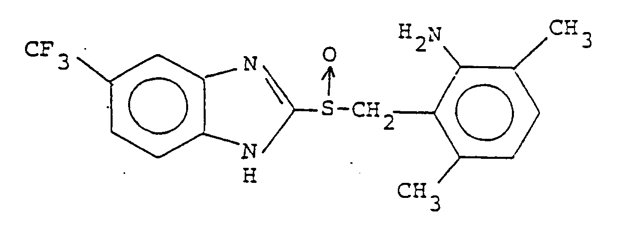

The title compound was prepared by the method of Example 3 using 578 mg of the title product of Example 28 instead of the title product of Example 2. The reaction mixture, which contained no precipitate, was washed with 5% aqueous potassium carbonate, dried over sodium sulfate, filtered, and concentrated in vacuo to a gum. Crystallization from acetonitrile gave 126 mg of the title compound.
Analysis. Calcd. for C₁₇H₁₆N₃FOS: C, 55.58; H, 4.39; N, 11.44; S, 8.73; F, 15.51.
Found: C, 55.43; H, 4.32; N, 11.48; S, 8.92; F, 15.31.

### Example 30 2-[(1H-benzimidazol-2-ylsulfinyl)methyl]-3,4,5-trimethylbenzenamine hemihydrate

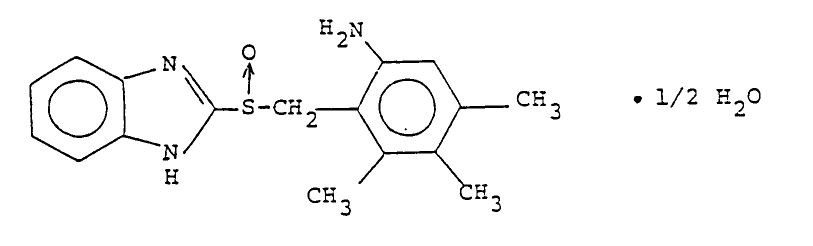

The title compound, m.p. 145° (softening) with decomp. at 235°, was prepared using 3,4,5-trimethylaniline and sulfuric acid during the reaction according to Example 2.
Analysis. Calcd. for C₁₇H₁₉N₃OS·H₂O: C, 63.33; H, 6.25; N, 13.03; F, 9.95.
Found: C, 63.53; H, 5.92; N, 13.16; F, 9.60.

### Example 31 2-[[(5-methoxy-1H-benzimidazol-2-yl)sulfinyl] methyl]-4-methoxy-3,5-dimethylbenzenamine

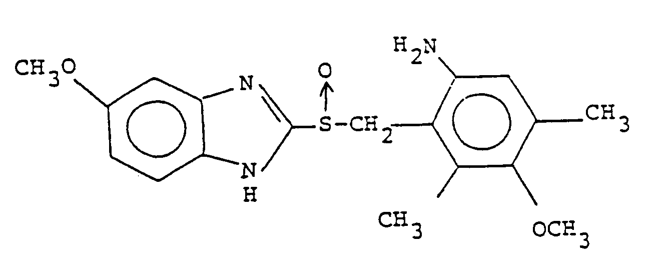

The title compound, m.p. 149-155°, was prepared using 2-mercapto-5-methoxybenzimidazole.
Analysis. Calcd. for C₁₈H₂₁N₃O₃S: C, 60.15; H, 5.89; N, 11.69; F, 8.92.
Found: C, 59.71; H, 5.83; N, 11.56; F, 8.64.

### Example 33

| Table of Pharmacological Test Results. | | | |
|---|---|---|---|
| Compound [Product of Example No.] | (H⁺+K⁺)-ATPase IC₅₀ (mcM) | Gastric-Fistula Beagle % Inhibition (3 mg/kg dose) | Pavlov-Pouch Dog % Inhibition (at dose (mg/kg)) |
| 3 | 4.3 | 59 i.v. | 87 (10 mg/kg) |
| | | 53 i.d. | 20 (1 mg/kg) |
| 5 | 11.0 | 90 i.d. | 54 (10 mg/kg) |
| | | | 19 (3 mg/kg) |
| 9 | 0.66 | 73 i.v. | |
| 11 | 2.1 | 67 i.v. | |
| 13 | 3.2 | 93 i.v. | 43 (3 mg/kg) |
| | | 44 i.d. | |
| 16 | 0.85 | | |
| 18 | 4.2 | 91 i.v. | 79 (3mg/kg) |
| | | 66 i.d. | |
| | | 43 i.g. | |
| 20 | 5.4 | 96 i.v. | |
| | | 72 i.d. | |
| 22 | 4.4 | 37 i.d. | |
| 26 | 0.82 | | |
| 28 | 5.3 | | |
| 30 | 1.5 | | |
| 31 | 16.0 | | |
| 31 | 2.1 | 31 i.d. | |

## Claims

1. 2-[[(1H-benzimidazol-2-yl)sulfinyl]methyl] - methylbenzene amines of the formula 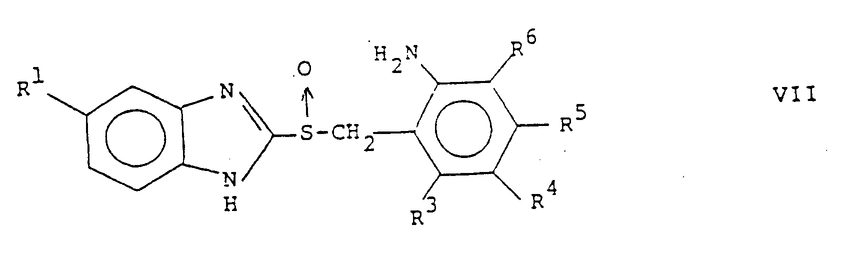 wherein R¹ is hydrogen, methoxy, ethoxy, or trifluoromethyl; and wherein at least one of R³, R⁴, R⁵, and R⁶ is methyl, the others being hydrogen or methoxy, and the pharmaceutical acceptable salts thereof.

2. 2-[[(1H-benzimidazol-2-yl)sulfinyl]methyl]-4-methylbenzene amines of the formula 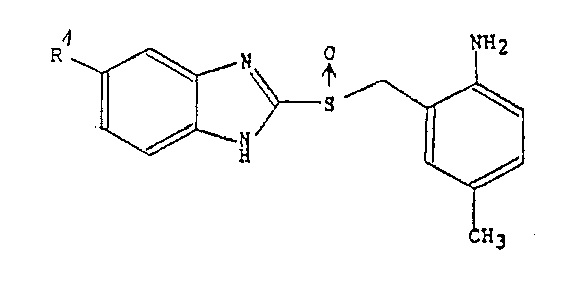 wherein R¹ is methoxy or ethoxy.

3. A pharmaceutical composition comprising at least one of the compounds of claim 1 or 2 together with one or more non-toxic pharmaceutically acceptable carriers.

4. A pharmaceutical composition according to claim 3 for treating ulcer in mammals.

## Patentansprüche

1. 2-[[(1H-Benzimidazol-2-yl)sulfinyl]methyl]-methyl-benzamine der Formel 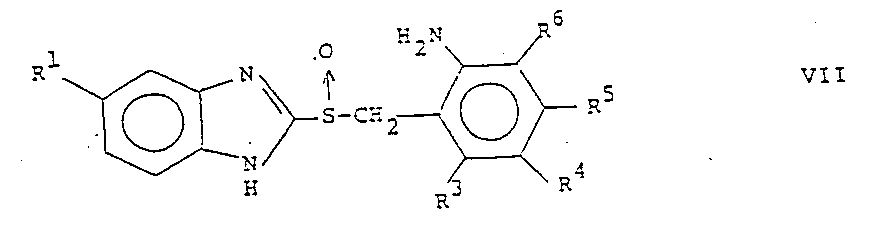 worin R¹ Wasserstoff, Methoxy, Ethoxy oder Trifluormethyl ist und worin wenigstens eine der Gruppen R³, R⁴, R⁵ und R⁶ Methyl ist, wobei die anderen Wsserstoff oder Methoxy sind, sowie deren pharmazeutisch verträglichen Salze.

2. 2-[[(1H-Benzimidazol-2-yl)sulfinyl]methyl]-4-methyl-benzamine der Formel 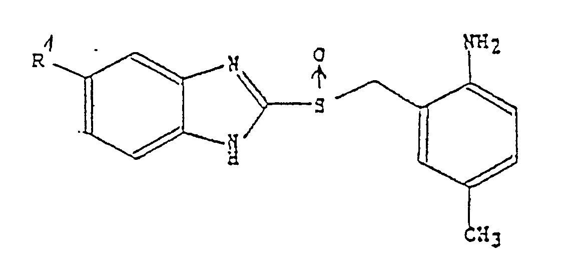 worin R¹ Methoxy oder Ethoxy ist.

3. Eine pharmazeutische Zusammensetzung, umfassend wenigstens eine Verbindung gemäß den Ansprüchen 1 oder 2 zusammen mit einem oder mehreren pharmazeutisch zulässigen nicht toxischen Trägern.

4. Eine pharmazeutische Zusammensetzung gemäß Anspruch 3 zur Behandlung von Geschwüren in Säugern.

## Revendications

1. 2-[[(1H-benzimidazole-2-yl)sulfinyl]méthyl]-méthylbenzèneamines de formules : 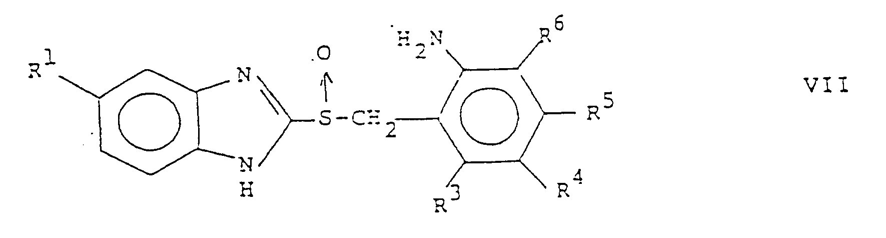 dans laquelle R¹ représente l'hydrogène, un groupe méthoxy, éthoxy, ou trifluorométhyle; et dans laquelle au moins l'un des R³, R⁴, R⁵ et R⁶ représente un groupe méthyle, et les autres étant de l'hydrogène ou un groupe méthoxy, ainsi que les sels pharmaceutiquement acceptables de ces amines.

2. 2-[[(1H-benzimidazole-2-yl)sulfinyl]méthyl]-4-méthylbenzèneamines de formule : 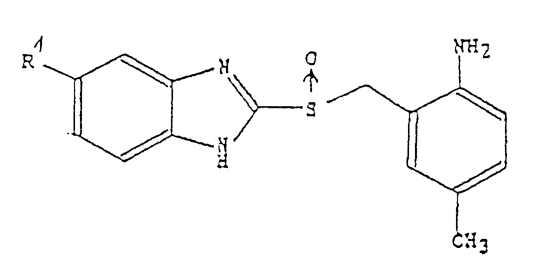 dans laquelle R¹ est un groupe méthoxy ou éthoxy.

3. Une composition pharmaceutique comprenant au moins un des composés selon la revendication 1 ou 2 ensemble avec un ou plusieurs supports non toxiques pharmaceutiquement acceptables.

4. Une composition pharmaceutique selon la revendication 3, pour le traitement de l'ulcère chez les mammifères.
